(19) Europäisches Patentamt
European Patent Office
Office européen des brevets

(11) Veröffentlichungsnummer: **0 150 374 B1**

(12) **EUROPÄISCHE PATENTSCHRIFT**

(45) Veröffentlichungstag der Patentschrift: **10.04.91**

(51) Int. Cl.⁵: **A61K 7/22**, A61K 31/14

(21) Anmeldenummer: **84115121.0**

(22) Anmeldetag: **10.12.84**

# (54) Mund- und Zahnpflegemittel.

(30) Priorität: **17.12.83 DE 3345781**

(43) Veröffentlichungstag der Anmeldung:
**07.08.85 Patentblatt 85/32**

(45) Bekanntmachung des Hinweises auf die Patenterteilung:
**10.04.91 Patentblatt 91/15**

(84) Benannte Vertragsstaaten:
**AT BE CH DE FR GB IT LI LU NL SE**

(56) Entgegenhaltungen:
**US-A- 3 277 118**
**US-A- 3 703 583**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Klüppel, Hans-Jürgen**
**Begonienstrasse 7**
**W-4000 Düsseldorf(DE)**
Erfinder: **Plöger, Walther, Dr.**
**Holbeinweg 11**
**W-4010 Hilden(DE)**
Erfinder: **Rutzen, Horst, Dr.**
**Falkenweg 12**
**W-4018 Langenfeld(DE)**
Erfinder: **Lehmann, Rudolf, Dr.**
**Schnugsheide 18**
**W-5653 Leichlingen 1(DE)**

**EP 0 150 374 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Mund- und Zahnpflegemittel mit einem Gehalt an belagverhindernden quartären Ammoniumverbindungen.

Mund- und Zahnpflegemittel sind Produkte, die der Reinigung und Pflege der Mundhöhle, der zähne und des Rachenraumes dienen. Die Aufgabe von Mund- und Zahnpflegemitteln ist neben der Reinigung der Zähne von Zahnbelägen, der sogenannten Zahnplaque, die Verhinderung der Bildung von Zahnstein und die Vorbeugung vor Zahnerkrankungen wie Karies und Parodontose sowie die Beseitigung von Mundgeruch.

Ein zentrales Problem ist die Entfernung der Zahnbeläge. Maßnahmen, welche die Plaquebildung verringern oder bereits gebildete Beläge wieder entfernen, führen zu einem Rückgang der Parodontopathien und auch zu einem Rückgang der Karies. Es ist daher ein Ziel der vorliegenden Erfindung, Mund- und Zahnpflegemittel mit einer gegen die Bildung von Zahnbelägen gerichteten Wirkung zu schaffen.

Ein bekannter Weg zur Verringerung der Plaquebildung ist der Einsatz von mikrobiziden Stoffen in Mund- und Zahn pflegemittel. Bekannte Beispiele sind das Chlorhexidin(1.1'-Hexamethylen-bis-[5-(4-chlorphenyl)-biguanid] und zahlreiche, antibakteriell wirksame quartäre Ammoniumverbindungen wie Cetylpyridiniumchlorid oder die aus der deutschen Auslegeschrift DE-AS 1.274.281 bekannten quartären Ammoniumverbindungen. Die Verwendung solcher antimikrobieller Stoffe birgt jedoch erhebliche Risiken, da diese ganz unspezifisch die gesamte Mikroflora der Mundhöhle angreifen. Dies kann Erkrankung der Mundhöhle verursachen oder fördern. Es ist auch bekannt, daß solche Mikrobizide zu erheblichen Schleimhautirritationen geführt haben. Ein weiterer Nachteil der meisten bekannten quartären Ammoniumverbindungen ist eine durch sie hervorgerufene Zahnverfärbung.

Aus der deutschen Auslegeschrift DE-AS 11 54 236 ist die Verwendung einer Vielzahl von quartären Ammoniumfluoriden zur Verringerung der Säurelöslichkeit des Zahnschmelzes bekannt. Die dort genannten Verbindungen sind entweder stark antimikrobiell und daher aus den genannten Gründen nicht brauchbar oder sie sind im Hinblick auf eine Belagsverhinderung nicht wirksam.

Es war daher ein weiteres Ziel der Erfindung, Stoffe aufzufinden, die in Mund- und Zahnpflegemitteln einerseits eine gute Wirkung gegen die Bildung von Zahnbelägen aufweisen, andererseits aber praktisch nicht antimikrobiell wirksam sind.

Überraschend wurde gefunden, daß Mund- und Zahnpflegemittel mit einem Gehalt an quartären Ammoniumverbindungen die gewünschten Eigenschaften aufweisen, wenn die quartären Ammoniumverbindungen der allgemeinen Formel I entsprechen,

$$\text{I}\quad \left[ R^1 - \underset{\displaystyle OH}{CH} - CH_2 - {}^{(+)}\overset{\displaystyle R^3}{\underset{\displaystyle CH_3}{N}} - CH_2 - \underset{\displaystyle OH}{CH} - R^2 \right] \frac{1}{n}\ A^{n(-)}$$

in der $R^1$ eine n-Alkylgruppe mit 8 - 12 C-Atomen, $R^2$ Wasserstoff, eine $CH_3$-Gruppe oder eine $HO\text{-}CH_2$-Gruppe und $R^3$ eine $CH_3$-Gruppe oder eine Gruppe $-CH_2\text{-}CH(OH)\text{-}R^2$, A ein anorganisches oder organisches Säureanion und n dessen Basizität darstellt.

Die quartären Ammoniumverbindungen der allgemeinen Formel I weisen in Mund- und Zahnpflegemitteln schon bei geringen Anwendungskonzentrationen eine ausgeprägte belagverhindernde Wirkung auf, welche die Wirkung der typischen antimikrobiellen Wirkstoffe, z.B. des Chlorhexidins, übertrifft. Andererseits sind die Verbindungen der allgemeinen Formel I nur sehr wenig und im Bereich der bereits gut plaqueinhibierenden Konzentrationen überhaupt nicht antimikrobiell wirksam. Bei klinischen Prüfungen wurden keine Zahnverfärbungen durch die quartären Ammoniumverbindungen der allgemeinen Formel I beobachtet.

Im Hinblick auf die Länge der Alkylgruppe $R^1$ liegt das anwendungstechnische Optimum bei solchen Verbindungen der allgemeinen Formel I, in welchen die Gruppe $R^1$ eine n-Decylgruppe ist. Mit steigender Kettenlänge von $R^1$ nimmt zwar die belagsverhindernde Wirkung, gleichzeitig aber auch die unerwünschte antimikrobielle Wirkung zu. Wenn $R^1$ ein Alkylrest mit weniger als 8 C-Atomen ist, fehlt zwar die antimikrobielle Wirkung, aber der belagverhindernde Effekt wird zu schwach. Die Gruppe $R^2$ ist bevorzugt Wasserstoff und $R^3$ eine Methylgruppe oder eine 2-Hydroxyethylgruppe, Bevorzugt geeignete Verbindungen der Formel I sind daher die 2-Hydroxydodecyl-2-hydroxyethyl-dimethylammonium-Salze und die 2-Hydroxydodecyl-di-(2-hydroxyethyl)-methylammonium-Salze.

Als Anion kann jedes physiologisch verträgliche, mit dem quartären Ammoniumion ein wasserlösliches Salz bildende anorganische oder organische Säureanion verwendet werden. Als anorganische Säureanionen kommen z.B. die Halogenide, insbesondere Fluorid, Chlorid oder Bromid, die Sulfate oder Hydrogensulfate, Phosphate, Hydrogenphosphate, Nitrate, Borate, Carbonat und Hydrogencarbonate in Frage. Als organische Säureanionen kommen z.B. Formiat, Acetat, Propionat, Lactat, Glykolat, Citrat, Tartrat, Malat, Malonat, Maleinat, Succinat, Gluconat, Benzoat, Salicylat, Sorbat, Ascorbat usw. in Frage. Besonders geeignet sind die Salze von Organophosphonsäuren, da die Organophosphonate z.B. gemäß US - PS 3.488.419, DE-OS 22 24 430 und DE-OS 23 43 196 selbst eine merkliche, zahnsteininhibierende Wirkung aufweisen.

Wegen der bekannten antikariogenen Eigenschaften des gelösten Fluorids sind Mund- und Zahnpflegemittel mit einem Gehalt an quartären Ammoniumverbindungen der Formel I, in welchen $A^{(-)}$ ein Fluoridanion darstellt, besonders bevorzugt. Eine weitere bevorzugte Ausführung der Erfindung sind Mund- und Zahnpflegemittel, die quartäre Ammoniumverbindungen der allgemeinen Formel I enthalten, in welchen A ein Organophosphonatanion, bevorzugt einer Organophosphonsäure der allgemeinen Formel II oder III enthalten ist,

II 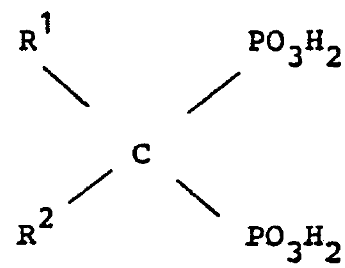

III 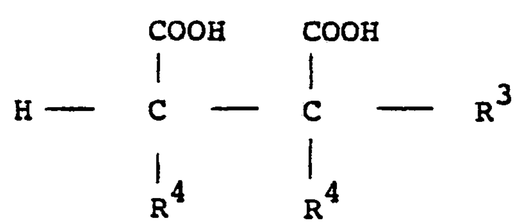

in welchen $R^1$ eine Alkylgruppe mit 1 - 6 C-Atomen, eine Hydroxylgruppe, eine Aminogruppe, eine Gruppe $-NHR^5$, in der $R^5$ eine Alkylgruppe mit 1 - 3 C-Atomen ist, eine Gruppe $-CH_2-COOH$, $-CH_2PO_3H_2$, $-CH_2-CH_2-PO_3H_2$, $-CH(PO_3H_2)$ (OH) oder $-CH_2-CH-(PO_3H_2)_2$ und $R^2$ Wasserstoff, eine Alkylgruppe mit 1 - 6 C-Atomen oder, wenn $R^1$ eine Aminogruppe oder eine Gruppe $-NHR^5$ ist, mit dem Stickstoffatom und dem Zentralkohlenstoffatom einen 5 - 7-gliedrigen Azacycloalkanring bildet, $R^3$ eine Gruppe $-PO_3H_2$, CR $(COOH)(PO_3H_2)$, $CR^4(PO_3H_2)_2$ oder $CR^4(COOH)$ $-CH_2-PO_3H_2$ und $R^4$ Wasserstoff oder eine Alkylgruppe mit 1 - 4 C-Atomen, insbesondere $-CH_3$ oder die Gruppe $-(CH_2)_{1-2}-COOH$ bedeutet.

Die quartären Ammoniumverbindungen der allgemeinen Formel I sind bekannt oder nach bekannten technischen Verfahren herstellbar. Die Herstellung kann z.B. nach dem von H. Rutzen in Fette, Seifen, Anstrichmittel 84 (1982), S. 87 - 92 beschriebenen Verfahren durch Quaternierung von tertiären Aminsalzen mit langkettigen 1.2-Epoxiden erfolgen. Nach dieser Methode lassen sich die erfindungsgemäß zu verwendenden quartären Ammoniumverbindungen,ausgehend von Salzen der allgemeinen Formel IV

IV 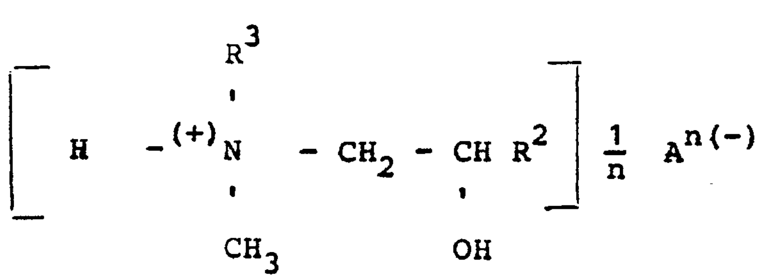

in der $R^2$,$R^3$, A und n die für die Formel I angegebene Bedeutung haben, durch Quaternierung mit 1.2-Epoxiden der Formel

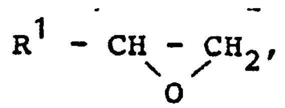

in der $R^1$ die für die Formel I angegebene Bedeutung hat, herstellen. Die wäßrigen Lösungen der Salze der allgemeinen Formel IV werden durch Umsetzung der tertiären Amine, z.B. von Dimethylethanolamin, Methyldiethanolamin, Dimethyl-2.3-dihydroxypropyl-amin mit anorganischen oder organischen Säuren, z.B. Salzsäure, Flußsäure, Schwefelsäure, Citronensäure, Glykolsäure, Milchsäure oder Organophosphonsäuren der allgemeinen Formel II oder III, z.B. 1-Hydroxyethan-1.1-diphosphonsäure oder 1-Phosphonopropan-1.2.3-tricarbonsäure in wäßriger Lösung hergestellt. Besonders vorteilhafte Reaktionsbedingungen für die Quaternierung werden z.B. in DE-OS 31 16 087 vorgeschlagen.

Die erfindungsgemäßen Mund- und Zahnpflegemittel mit einem Gehalt an quartären Ammoniumverbindungen der allgemeinen Formel I können in den verschiedenen für solche Produkte üblichen Zubereitungsarten, z.B. als Mundwässer, Zahnpasten oder Zahnpulver hergestellt werden. Der Gehalt an quartären Ammoniumverbindungen sollte im Bereich von 0,01 % bis 2,0 % der Zubereitung liegen. Zur Erzielung einer befriedigenden Belagsverhinderung sind in unverdünnt anzuwendenden Mundwässern Konzentrationen von 0,03 - 0,3 Gew.-%, in Zahnpasten Konzentrationen von 0,1 - 0,5 Gew.-% der gesamten Zubereitung besonders bevorzugt. In Mundwasserkonzentraten, die vor Anwendung verdünnt werden, sind entsprechend dem vorgesehenen Verdünnungsverhältnis höhere Konzentrationen einzusetzen.

Mund- und Zahnpflegemittel können darüber hinaus auch in der Form von Kaugummi, Mundpastillen und Zahnbehandlungssalben zubereitet werden. Solchen Mundpflegemitteln, die gegebenenfalls mehrmals täglich angewandt und zwangsläufig verschluckt werden, können ebenfalls quartäre Ammoniumverbindungen der Formel I zugesetzt werden. In diesen Fällen sollte die Dosierung der quartären Ammoniumverbindungen jedoch nicht über 0,1 Gew.-% der Zubereitung hinausgehen.

Neben den quartären Ammoniumverbindungen können die erfindungsgemäßen Mund- und Zahnpflegemittel die für die jeweilige Zubereitungsform üblichen Träger- und Zusatzmittel enthalten.

In Mundwässern ist eine Kombination mit den wäßrigalkoholischen Lösungen verschiedener Grädigkeit von ätherischen Ölen, Emulgatoren, adstringierenden und tonisierenden Drogenauszügen, karieshemmenden Zusätzen und Geschmackskorrigentien ohne weiteres möglich. Bei der Auswahl der zu verwendenden Emulgatoren und Netzmittel sind nichtionogene und ampholytische oder zwitterionische Tenside bevorzugt, da es mit anionischen Tensiden, z.8. mit Seifen und Alkylsulfaten zu unerwünschten Wechselwirkungen zwischen diesen und den quartären Ammoniumverbindungen kommen kann, welche die belagsverhindernde Wirkung stören. Bevorzugt zu verwenden sind nichtionogene Emulgatoren und Netzmittel, z.B. ethoxylierte Sorbitanfettsäureester, ethoxylierte Glycerinfettsäureester, Alkylglucoside, Fettalkoholpolyglycolester, Ethylenoxid-propylenoxid-Blockpolymerisate und Aminoxide. Schließlich ist zu berücksichtigen, daß die erfindungsgemäß einzusetzenden quartären Ammoniumverbindungen selbst Tenside sind.

Unter Zahnpasten oder Zahncremes werden im allgemeinen pastöse Zubereitungen aus Wasser, Verdickungsmitteln, Feuchthaltemitteln, Schleif- oder Putzkörpern, Tensiden, Süßungsmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z.B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden. Wenn die erfindungsgemäß zu verwendenden quartären Ammoniumverbindungen in Form der Salze von Organophosphonsäuren zum Einsatz kommen, empfiehlt sich der Einsatz von Putzkörpern, die frei von löslichem Calcium sind, damit die zahnsteinverhütende Wirkung der Organophosphonate nicht beeinträchtigt wird.

Bevorzugt geeignete Putzkörper für die erfindungsgemäßen Zahnpasten sind vor allem feinteilige Xerogel-Kieselsäuren, Hydrogelkieselsäuren, Fällungskieselsäuren, Aluminiumoxid-trihydrat und feinteiliges $\alpha$ -Aluminiumoxid oder Mischungen dieser Putzkörper in Mengen von 15 - 40 Gew.-% der Zahnpaste. Als Feuchthaltemittel kommen vorwiegend niedermolekulare Polyethylenglykole, Glycerin, Sorbit oder Mischungen dieser Produkte in Mengen bis ca. 50 Gew.-% in Frage. Unter den bekannten Verdickungsmitteln sind die verdickenden, feinteiligen Gelkieselsäuren und die nichtionogenen Hydrocolloide, wie z.B. Hydroxyethylcellulose, Hydroxypropylguar, Hydroxyethylstärke, Polyvinylpyrrolidon, hochmolekulares Polyethylenglykol, Pflanzengumme wie Traganth, Agar-Agar, Carragheenmoos, Gummiarabicum und Xantham-Gum bevorzugt geeignet. Anionische Hydrocolloide können mit den quartären Ammoniumverbindungen in Wechselwirkung treten, so daß deren belagsverhindernde Wirkung gestört wird.

Obwohl die quartären Ammoniumverbindungen der Formel I bereits selbst oberflächenaktiv sind, können weitere Tenside, bevorzugt aus der Gruppe der schon genannten nichtionogenen Tenside, aber auch zwitterionische und amphotere Tenside den Zahnpasten zugesetzt werden.

Die erwünschte Aroma- und Geschmacksnote läßt sich durch die dafür gebräuchlichen ätherischen Öle, wie z.B. Pfefferminze , Nelken-, Wintergrün-, Sassafrasöl sowie durch Süßungsmittel wie z.B. Saccharin, Cyclamat, Dulcin, Dextrose, Lävulose usw. erzielen. Wenn die erfindungsgemäß zu verwendenden quartären Ammoniumverbindungen in den Zahnpflegemitteln nicht bereits in der Form von Fluoriden oder Organophosphonaten enthal ten sind, ist es auch möglich, diese antikariogen wirksamen Stoffe zusätzlich in der Form von z.B.Alkalifluoriden, Alkali-monofluorphosphaten oder der Alkalisalze von Organophosphonsäuren, insbesondere solchen der allgemeinen Formel II und III den Mund- und Zahnpflegemitteln zuzusetzen.

Darüber hinaus können die erfindungsgemäßen Mund- und Zahnpflegemittel weitere übliche Hilfsmittel, z.B. Farbstoffe, Trübungsmittel, z.B. Titandioxid, Konservierungsstoffe enthalten.

Die folgenden Versuche und Anwendungsbeispiele sollen den Gegenstand der Erfindung näher erläutern.

1. Herstellung von 2-Hydroxydodecyl-2-hydroxyethyldimethyl-ammonium-fluorid

44,57 g Dimethylethanolamin (0,5 Mol) wurden mit 420,65 g Wasser verdünnt und mit 19,38 g einer wäßrigen, 41,3 %igen Flußsäurelösung (0,4 Mol) versetzt. Es entstand eine klare Lösung. Nach Erwärmen der Lösung auf 95° C wurden 94,0 g 1.2-Epoxydodecan (0,5 Mol) und 3,5 g einer 75 %igen Dispersion von Distearyldimethylammoniumchlorid (als Phasentransferkatalysator) zugesetzt. Dann wurde das Reaktionsgemisch 6 Std. bei 95° C gerührt. Zuletzt wurden noch 4,84 g der 41,3 %igen Flußsäurelösung (0,1 Mol) zugesetzt. Es wurde eine klare gelbe Lösung des 2-Hydroxydodecyl-2-hydroxyethyl-dimethylammoniumfluorids mit einem Gehalt von 73,1 m•val quartärer Ammoniumverbindung (QAV) pro 100 g der wäßrigen Lösung erhalten.

2. Herstellung von 2-Hydroxydodecyl-bis-(2-hydroxyethyl)-methylammonium-fluorid

Nach dem Verfahren analog Beispiel 1 wurde aus
66,5 g Methyl-diethanolamin (98,5 %ig)
24,2 g wäßrige Flußsäure (41,3 %ig)
94,0 g 1.2-Epoxydodecan
137,0 g Wasser
7,0 g Distearyldimethylammoniumchlorid (75 %ig)
in einer Reaktionszeit von 19 Std. bei 95° C eine leicht getrübte Lösung aus 2-Hydroxydodecyl-di-(2-hydroxyethyl)-methylammoniumfluorid mit einem Gehalt von 146,8 m•val quartärer Ammoniumverbindung (QAV) pro 100 g der wäßrigen Lösung erhalten.

3. Herstellung von -Tris-[2-Hydroxydodecyl-2-hydroxyethyldimethylammonium]-1-hydroxyethan-1,1-diphosphonat

49,02 g Dimethylethanolamin (0,55 Mol) wurden mit 483,85 g Wasser verdünnt und mit 57,35 g einer 60 %igen wäßrigen Lösung von 1-Hydroxyethan-1.1-diphosphonsäure (0,167 Mol) versetzt. Nach Zugabe von 94.0 g 1.2-Epoxydodecan (0,5 Mol) und 3,5 g einer 75 %igen Dispersion von Distearyldimethylammoniumchlorid wurde das Reaktionsgemisch auf 95° C erwärmt und 6 Std. gerührt. Nach dieser Zeit war eine klare gelbe Lösung mit einem Gehalt von 72,0 m•val 2-Hydroxydodecyl-2-hydroxyethyl-dimethylammonium-1-hydroxyethan-1.1-diphosphonat pro 100 g Lösung entstanden.

4. Herstellung von 2-Hydroxydodecyl-2-hydroxyethyldimethylammoniumchlorid

382,03 g Dimethylethanolamin (4,28 Mol) wurden mit 1046.19 g Wasser und 805,76 g 1.2-Epoxydodecan (4,28 Mol) vorgelegt und 338,03 g einer wäßrigen 37 %igen Salzsäurelösung unter einer Stickstoffatmosphäre zugetropft. Anschließend wurde die Lösung auf 90° C im Wasserbad unter Rühren erwärmt. Nach 1 Stunde bildeten sich 2 Phasen aus, nach weiteren 20 Minuten stieg die Viskosität des Reaktionsgemisches

merklich an. Nach 2,5 Stunden Rühren betrug die Epoxidzahl des Reaktionsgemisches 0,05 (Gew.-% Epoxid-Sauerstoff). Danach wurden weitere 84,51 g der 37 %igen Salzsäurelösung zugegeben, wo durch das Reaktionsgemisch wieder dünnflüssig wurde.

Nach Zugabe von 665 ml Toluol, 665 ml Cyclohexan und 55o ml Isopropanol wurde das Wasser mit den Lösungsmitteln azeotrop abdestilliert. Letzte Reste von Wasser und Lösungsmitteln wurden im Wasserstrahlvakuum entfernt. Der Rückstand wurde aus Azeton umkristallisiert. Es wurden 978 g eines farblosen Produktes mit einem Gehalt von 295,7 m•val quartärer Ammoniumverbindung (QAV) pro 100 g Produkt erhalten.

5. Vergleich der antibakteriellen Eigenschaften zwischen

A : Verbindung nach Beispiel 4
B : Verbindung nach Beispiel 3
C : Chlorhexidin
D : Cetylpyridiniumchlorid

Von den genannten Wirkstoffen wurde die Abtötungskinetik gegenüber einem typischen, kariogenen Keim, Streptococcus mutans, mit Hilfe eines quantitativen Suspensionstestes in folgender Weise ermittelt:

Die Verbindungen A, B, C und D wurden in demineralisiertem Wasser gelöst (bzw. wäßriger Lösungen entsprechend verdünnt), so daß die Produkte in einer Wirkstoffkonzentration von 500 ppm vorlagen.

Jeweils 10 ml dieser Wirkstofflösungen wurden mit 0,1 ml einer Testkeimsuspension, die 2 x $10^8$ Keime pro ml enthielt, beimpft, und bei Raumtempera tur (20 °C) gehalten. Aus diesen Testansätzen wurden nach Einwirkzeiten von 0,5 Minuten, 1 Minute und 2 Minuten Proben von je 0,1 ml entnommen und sowohl direkt als auch nach Verdünnung mit demineralisiertem Wasser im Verhältnis von 1 : 10 auf Agar-Nährböden ausgespatelt.

Zur Neutralisierung des Wirkstoffes nach der Einwirkungszeit enthielt sowohl das Verdünnungsmedium als auch der Nähr-Agar jeweils ein Gemisch aus 3 Gew.-% Tween(R) 80 und 0,3 Gew.-% Lecithin.

Nach 2-tägiger Bebrütung bei 37 °C wurden die bewachsenen Nährböden ausgezählt und daraus die Anzahl der überlebenden Zellen berechnet (Abb. 1 und 2).

Abb. 1
Abtötung von Streptococcus mutans
(Konzentration der Wirkstoffe 500 ppm)

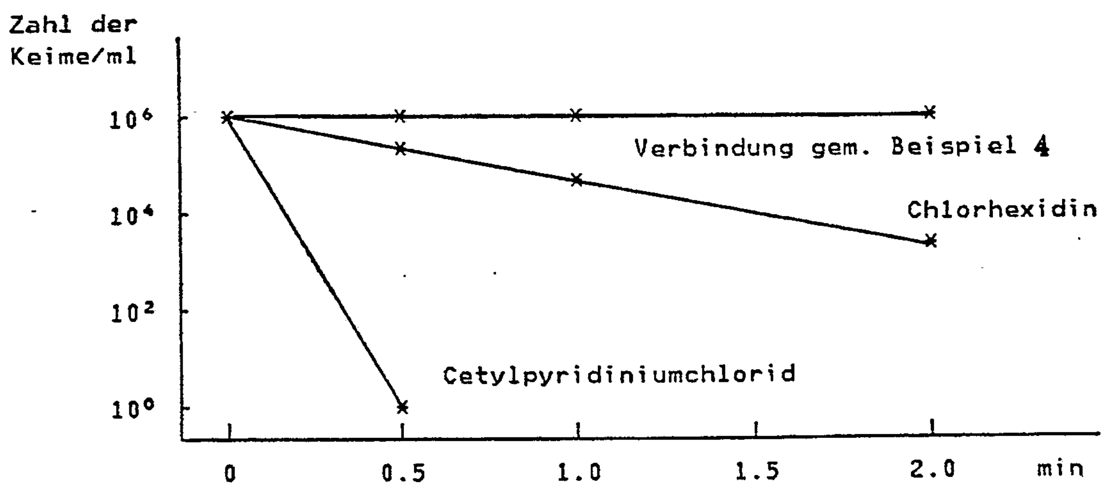

Abb. 2
Abtötung von Streptococcus mutans
(Konzentration der Wirkstoffe 500 ppm)

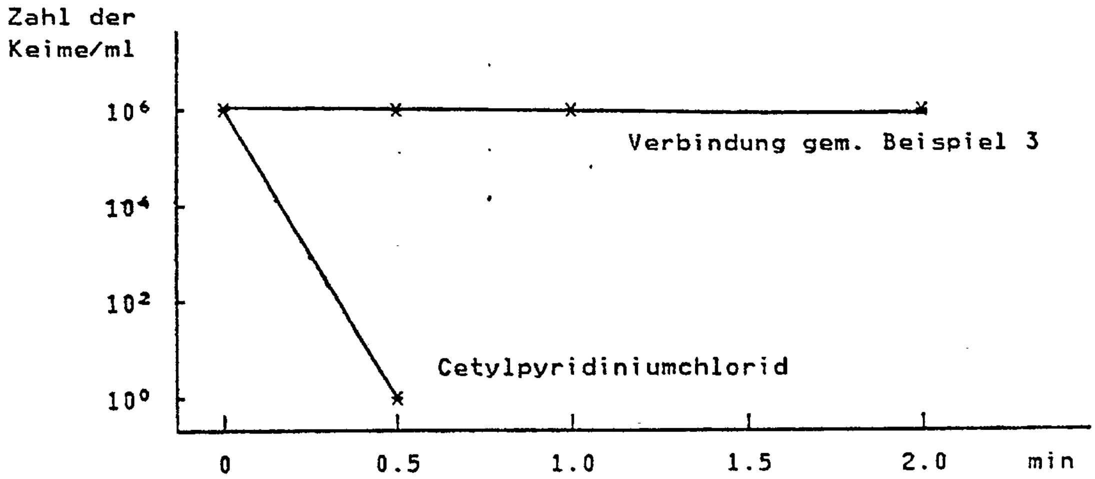


Die Versuche beweisen, daß durch die erfindungsgemäß zu verwendenden Verbindungen in praxisrelevanten Zeiten keine Abtötung oder Hemmung von Streptococcus mutans, einem wichtigen Karieskeim, erreicht wird.

6. Klinische Prüfung der Hemmung des Plaque-Wachstums durch Verwendung eines Mundwassers

a) ohne Wirkstoff
b) mit 0,1 Gew.-% Chlorhexidin
c) mit 0,1 Gew.-% der Verbindung nach Beispiel 1

An der Prüfung nahmen 24 Probanden teil, deren Plaque-Wachstum durch intensives Zähneputzen auf ein gleich niedriges Niveau gebracht worden war. Dann wurden die Probanden in 3 Gruppen zu je 8 Personen eingeteilt und jeder der drei Gruppen erhielt ein Mundwasser der folgenden Zusammensetzung zur 3 x täglichen Anwendung:

| Zusammensetzung Gew.-% | Gruppe 3 (Placebo) | Gruppe 2 | Gruppe 1 |
|---|---|---|---|
| Chlorhexidin | - | 0,1 | - |
| Verbindung Beispiel 1 (wasserfrei) | - | - | 0,1 |
| Ethanol | 5,0 | 5,0 | 5,0 |
| Saccharin-Na | 0,01 | 0,01 | 0,01 |
| Aroma | 1,0 | 1,0 | 1,0 |
| Wasser (schwach blau gefärbt) | ad 100 | ad 100 | ad 100 |

Alle mechanischen Mundhygienemaßnahmen unterblieben während der Testdauer von 4 Tagen. Das

Wachstum der Zahnplaque wurde jeden Morgen nach der Methode von Harrap (Journal of Clinical Periodontology 1974, 1 , S. 166 - 174) in Form des "Marginal Line Plaque Index" vermessen.

Das Ergebnis dieser Prüfung ist in der Abbildung 3 dargestellt.

Eine Fleckenbildung durch die erfindungsgemäße Verbindung wurde während des Kliniktests von 4 Tagen, aber auch bei längerer Verwendung, nicht beobachtet.

Eine Beeinträchtigung der plaqueinhibierenden Wirkung durch die im Speichel vorhandenen Proteine wurde nicht beobachtet.

Abb. 3
Klinische Prüfung zur Inhibierung des Plaquewachstums

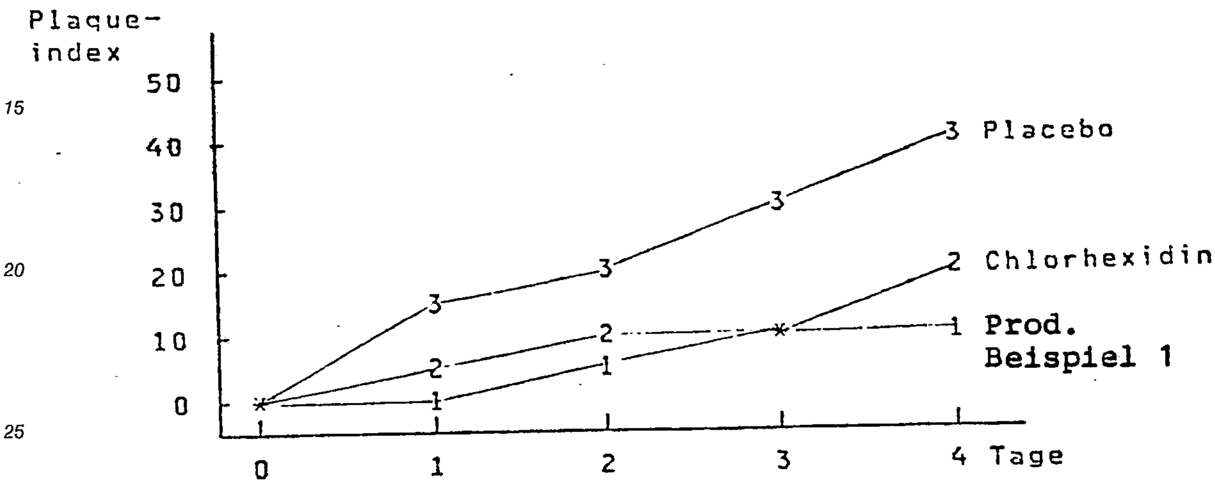


Die Prüfung der Verbindung gemäß Beispiel 3 erbrachte das gleiche Ergebnis.

7. Anwendungsbeispiele

### 7.1 Mundwasser

| | |
|---|---|
| Ethylalkohol 96 Vol % | 10,0 Gew.-% |
| Tween[(R)] 20 | 0,4 Gew.-% |
| Aromaöl | 0,3 Gew.-% |
| Sorbit (70 %ige wäßrige Lösung) | 8,0 Gew.-% |
| p-Hydroxybenzoesäure-methylester | 0,16 Gew.-% |
| Verbindung gemäß Beispiel 4 | 0,05 Gew.-% |
| Saccharin-Natrium | 0,1 Gew.-% |
| Farbstoff q.s. | |
| Wasser (demineralisiert) | ad 100 Gew.-% |

| Zahnpasten | 7.2 | 7.3 | |
|---|---|---|---|
| Xerogel-Kieselsäure (Syloblanc(R) 31) | 14 | 14 | Gew.-% |
| Aerogel-Kieselsäure (Syloid(R) 244) | 6 | 6 | " " |
| Sorbit (70 %ige wäßrige Lösung) | 25 | 25 | " " |
| Glycerin (86 %ige wäßrige Lösung) | 15 | 15 | " " |
| Polyethylenglykol 400 | 2 | 4 | " " |
| Hydroxyethylcellulose (Cellosize(R) WP 300) | 0,6 | 0,45 | " " |
| Fettalkoholpolyglykolether (Dehydol(R) TA 25) | 2,5 | 2,5 | " " |
| Verbindung gemäß Beispiel 4 | 0,3 | 0,3 | " " |
| Natriumfluorid | 0,22 | - | " " |
| 1-Hydroxyethan-1.1-Diphosphonsäure-dinatriumsalz | - | 1,23 | " " |
| Titandioxid | 0,8 | 0,8 | " " |
| p-Hydroxybenzoesäuremethylester | 0,16 | 0,16 | " " |
| Aromaöl | 1,0 | 1,0 | " " |
| Saccharin-Natrium | 0,2 | 0,2 | " " |
| Wasser (demineralisiert) | ad 100 | ad 100 | " " |

## Ansprüche

1. Mund- und Zahnpflegemittel mit einem Gehalt an quartären Ammoniumverbindungen und den für die jeweilige Zubereitungsform üblichen Träger und Zusatzmitteln, dadurch gekennzeichnet, daß die quartären Ammoniumverbindungen der allgemeinen Formel I entsprechen,

I 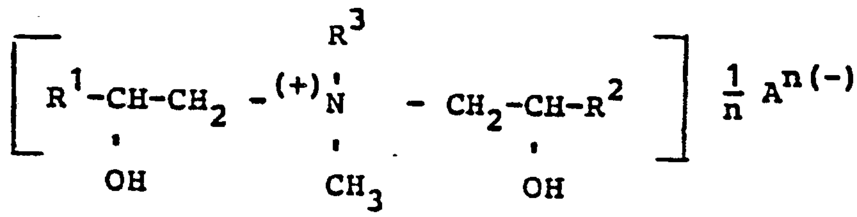

in der $R^1$ eine n-Alkylgruppe mit 8 - 12 C-Atomen, $R^2$ Wasserstoff, eine $Ch_3$-Gruppe oder eine $-CH_2OH$-Gruppe und $R^3$ eine $CH_3$-Gruppe oder eine Gruppe $-CH_2-CH(OH)-R^2$, A ein anorganisches oder organisches Säureanion und n dessen Basizität darstellt.

2. Mund- und Zahnpflegemittel nach Anspruch 1, dadurch gekennzeichnet, daß in der Formel I $R^1$ eine n-Decylgruppe darstellt.

3. Mund- und Zahnpflegemittel nach Anspruch 1 oder 2, dadurch gekennzeichnet, daß in der Formel I $R^2$ Was-serstoff und $R^3$ eine $CH_3$-Gruppe oder eine 2-Hydroxyethylgruppe darstellt.

4. Mund- und Zahnpflegemittel nach Anspruch 1 - 3, dadurch gekennzeichnet, daß in der Formel I A ein Fluoridanion darstellt.

5. Mund- und Zahnpflegemittel nach Anspruch 1 - 3, dadurch gekennzeichnet, daß A ein Organophosphonatanion, bevorzugt einer Organophosphonsäure der allgemeinen Formel II oder III ist

II 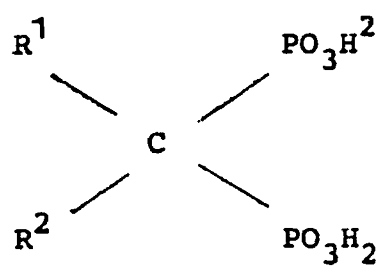

III 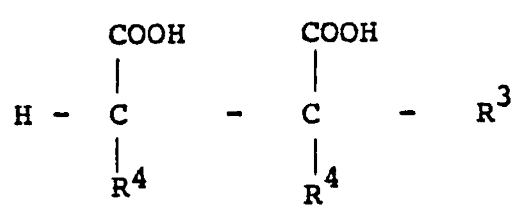

in welchen $R^1$ eine Alkylgruppe mit 1 - 6 C-Atomen, eine Hydroxylgruppe, eine Aminogruppe, eine Gruppe $-NHR^5$, in der $R^5$ eine Alkylgruppe mit 1 - 3 C-Atomen ist, eine Gruppe $-CH_2-COOH$, $-CH_2-PO_3H_2$, $-CH_2-CH_2-PO_3H_2$, $-CH(PO_3H_2)$ (OH) oder - $CH_2-CH(PO_3H_2)_2$ und $R_2$ Wasserstoff, eine Alkylgruppe mit 1 - 6 C-Atomen oder, wenn $R^1$ eine Aminogruppe oder eine Gruppe $NHR^5$ ist, mit dem

Stickstoffatom und dem Zentralkohlenstoffatom einen 5 -7-gliedrigen Azacycloalkanring bildet, $R^3$ eine Gruppe $-PO_3H_2$, $CR^4(COOH)(PO_3H_2)$, $CR^4(PO_3H_2)_2$ oder $CR^4(COOH)-CH_2-PO_3H_2$ und $R^4$ Wasserstoff oder einen Alkylrest mit 1 - 4 C-Atomen, insbesondere $-CH_3$ oder die Gruppe $-(CH_2)_{1-2}-COOH$ bedeutet.

6. Mundwasser in Form einer unverdünnt anwendbaren wäßrigen oder wäßrig-alkoholischen Lösung mit üblichen Geschmacks-und Aromazusätzen, dadurch gekennzeichnet, daß quartäre Ammoniumverbindungen der allgemeinen Formel I gemäß Anspruch 1 - 5 in einer Menge von 0,03 bis 0,3 Gew.-% der gesamten Zubereitung und gegebenenfalls nichtionogene oberflächenaktive Stoffe enthalten sind.

7. Zahnpasten in Form einer pastösen Zubereitung aus Wasser, Verdickungsmitteln, Feuchthaltemitteln, Schleif- und Putzkörpern, Geschmacks- und Aromazusätzen, deren Schleif- und Putzkörper ganz oder überwiegend aus feinteiligen Xerogel-Kieselsäuren, Hydrogelkieselsäuren, Fällungskieselsäuren, Aluminiumoxid-trihydrat und/oder feinteiligem $\alpha$ -Aluminiumoxid besteht, dadurch gekennzeichnet, daß quartäre Ammoniumverbindungen der allgemeinen Formel I gemäß Anspruch 1 - 4 in einer Menge von 0,1 - 0,5 Gew.-% der gesamten Zubereitung enthalten sind.

## Claims

1. Oral and dental hygiene preparations containing quaternary ammonium compounds and the supports and additives typical of the particular formulation, characterized in that the quaternary ammonium compounds correspond to the following general formula

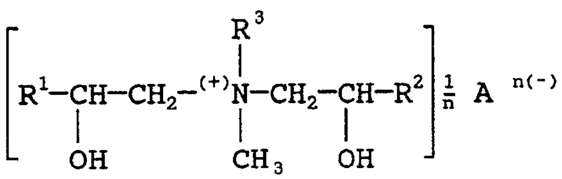

(I)

in which $R^1$ is an n-alkyl group containing from 8 to 12 carbon atoms, $R^2$ is hydrogen, a $CH_3$-group or a $CH_2OH$-group and $R^3$ is a $CH_3$-group or a group of the formula $-CH_2-CH(OH)-R^2$, A is an inorganic or organic acid anion and N represents its basicity.

2. Oral and dental hygiene preparations as claimed in claim 1, characterized in that, in formula I, $R^1$ is an n-decyl group.

3. Oral and dental hygiene preparations as claimed in claim 1 or 2, characterized in that, in formula I, $R^2$ represents hydrogen and $R^3$ is a $CH_3$-group or a 2-hydroxyethyl group.

4. Oral and dental hygiene preparations as claimed in claims 1 to 3, characterized in that, in formula I, A is a fluoride anion.

5. Oral and dental hygiene preparations as claimed in claims 1 to 3, characterized in that A is an organophosphonate anion, preferably of an organophosphonic acid corresponding to general formula II or III below

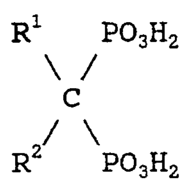

(II)

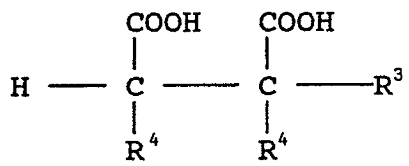

(III)

in which $R^1$ is an alkyl group containing from 1 to 6 carbon atoms, a hydroxyl group, an amino group, a group of the formula $-NHR^5$ where $R^5$ is an alkyl group containing from 1 to 3 carbon atoms, a group of the formula $-CH_2-COOH$, $-CH_2PO_3H_2$, $-CH_2-CH_2-PO_3H_2$, $-CH(PO_3H_2)(OH)$or $-CH_2-CH-(PO_3H_2)_2$ and $R^2$ is hydrogen, an alkyl group containing from 1 to 6 carbon atoms or, where $R^1$ is an amino group or an $-NHR^5$ group, forms a 5- to 7-membered azacycloalkane ring with the nitrogen atom and the central carbon atom, $R^3$ is a group of the formula $-PO_3H_2$, $CR^4(COOH)(PO_3H_2)$,$CR^4$ $(PO_3H_2)_2$ or $CR^4$ COOH)-$CH_2-PO_3H_2$ and $R^4$ is hydrogen or an alkyl group containing from 1 to 4 carbon atoms, more particularly $-CH_3$ or the group $-(CH_2)_{1-2}-COOH$.

6. A mouthwash in the form of an aqueous or aqueous-alcoholic solution containing the usual flavour and aroma additives, the mouthwash being useable without dilution, characterized in that quaternary ammonium compounds corresponding to general formula I as defined in claims 1 to 5 are present in a quantity of from 0.03 to 0.3% by weight, based on the preparation as a whole, optionally together with nonionic surfactants.

7. Toothpastes in the form of a paste-like preparation of water, thickeners, humectants, abrasives and polishes, flavour and aroma additives, of which the abrasives and polishes consist entirely or predominantly of finely particulate xerogel silicas, hydrogel silicas, precipitated silicas, aluminium oxide trihydrate and/or finely particulate $\alpha$-aluminium oxide, characterized in that quaternary ammonium compounds corresponding to general formula I as defined in claims 1 to 4 are present in a quantity of from 0.1 to 0.5% by weight, based on the preparations as a whole.

## Revendications

1. Produits d'hygiène bucco-dentaire contenant des composés d'ammonium quaternaire, ainsi que les vecteurs et additifs usuels pour chaque forme de préparation, caractérisés en ce que les composés d'ammonium quaternaire correspondent à la formule générale I,

I 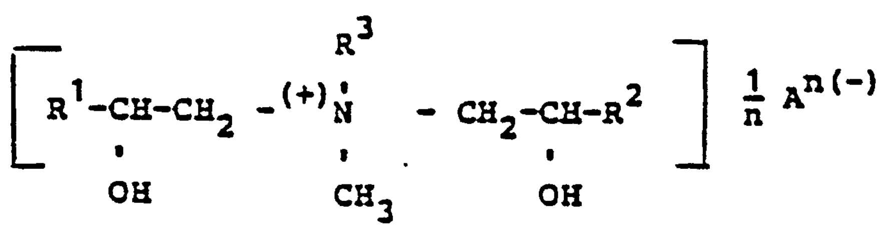

dans laquelle $R^1$ est un groupement n-alkyle comportant 8 à 12 atomes de C,$R^2$ représente l'hydrogène, un groupement $-CH_3$ ou un groupement $-CH_2$ OH, et $R^3$ correspond à un groupement $-CH_3$ ou à un groupement $-CH_2-CH(OH)-R^2$, A est un anion d'acide inorganique ou organique et n représente sa basicité.

2. Produits d'hygiène bucco-dentaire selon la revendication 1, caractérisés en ce que dans la formule I, $R^1$ représente un groupement n-décyle.

3. Produits d'hygiène bucco-dentaire selon la revendication 1 ou 2, caractérisés en ce que dans la formule I, $R^2$ représente l'hydrogène et $R^3$ correspond à un groupement $-CH_3$ ou à un groupement 2-hydroxy-éthyle.

4. Produits d'hygiène bucco-dentaire selon les revendications 1 à 3, caractérisés en ce que dans la

formule I, A représente un anion fluorure.

**5.** Produits d'hygiène bucco-dentaire selon les revendications 1 à 3, caractérisés en ce que A est un anion organophosphonate, de préférence celui d'un acide organophosphonique des formules générales II ou III

II

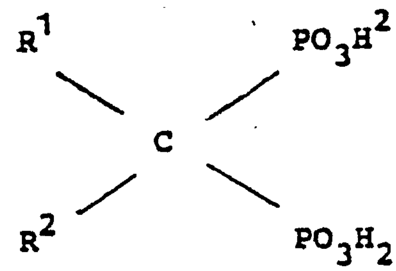

III

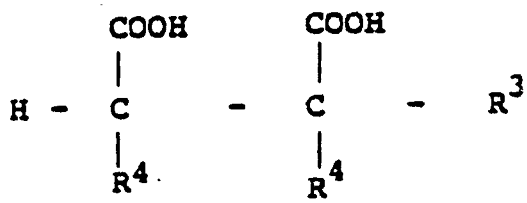

dans lesquelles $R^1$ représente un groupement alkyle comportant 1 à 6 atomes, un groupement hydroxyle, un groupement amino, un groupement -$NHR^5$, dans lequel $R^5$ est un groupe alkyle comportant 1 à 3 atomes de C, un groupe $-CH_2-COOH$,$-CH_2PO_3H_2$,$-CH_2-CH_2-PO_3H_2$,$-CH(PO_3H_2)$ (OH) ou $-CH_2-CH-(PO_3H_2)_2$ et $R^2$ représente l'hydrogène, un groupement alkyle comportant 1 à 6 atomes de C ou, si $R^1$ est un groupement amino ou un groupement -NHR , forme avec l'atome d'azote et l'atome central de carbone un noyau azacycloalcane comportant 5 à 7 chaînons, $R^3$ représente un groupement $-PO_3H_2$, $CR^4(COOH)(PO_3H_2)$, $CR^4(PO_3H_2)_2$ ou $CR^4(COOH)$ $-CH_2-PO_3H_2$ et $R^4$ est l'hydrogène ou un radical alkyle comportant 1 à 4 atomes de C, en particulier, $-CH_3$ ou le groupe $-(CH_2)_{1-2}-COOH$.

**6.** Collutoires sous la forme d'une solution aqueuse ou aqueuse-alcoolique utilisable sans dilution et renfermant les additifs gustatifs et aromatiques usuels, caractérisés en ce qu'ils contiennent des composés d'ammonium quaternaire de la formule générale I, selon les revendications 1 à 5, dans des proportions comprises dans l'intervalle de 0,03 à 0,3 % en poids par rapport à l'ensemble de la préparation et, le cas échéant, des agents de surface non ioniques.

**7.** Pâtes dentifrices sous la forme d'une préparation pâteuse constituée d'eau, d'épaississants, d'humidifiants, de produits abrasifs et nettoyants, d'additifs gustatifs et aromatiques, dont les agents abrasifs et nettoyants sont constitués en totalité ou en majeure partie d'acides siliciques en xérogel, d'acides siliciques en hydrogel, d'acides siliciques précipités, de trihydrate d'oxyde d'aluminium et/ou d'oxyde d'alpha-aluminium à fines particules, caractérisées en ce qu'elles contiennent des composés d'ammonium quaternaire de la formule générale I, selon les revendications 1 à 4, dans des proportions comprises dans l'intervalle de 0,1 à 0,5 % en poids par rapport à l'ensemble de la préparation.